# EUROPEAN PATENT SPECIFICATION

(11) **EP 1 450 859 B1**
(45) Date of publication and mention of the grant of the patent: **25.04.2012**
(21) Application number: 02795780.2
(22) Date of filing: 09.12.2002
(51) Int. Cl.: A61K 39/395, A61K 49/00, G01N 33/50, G01N 33/53, A61K 31/221

(54) **METHODS AND COMPOSITIONS FOR THE DIAGNOSIS OF ASTHMA**
VERFAHREN UND ZUSAMMENSETZUNGEN ZUR DIAGNOSE VON ASTHMA
METHODES ET COMPOSITIONS PERMETTANT DE DIAGNOSTIQUER L'ASTHME

(30) Priority: 07.12.2001 US 338389 P
(43) Date of publication of application: 01.09.2004
(73) Proprietor: University of Wyoming, Laramie, WY 82071 (US)
(72) Inventor: BROUGHTON, Kenneth, Shane, Laramie, WY 82070 (US); DRISKILL, John Gordon, Laramie, WY 82071 (US)
(74) Representative: Forrest, Graham Robert
(86) International application number: PCT/US2002/039198
(87) International publication number: WO 2003/049599

(56) References cited:
- US-A- 4 366 241
- US-A- 6 156 503
- DRAZEN J M ET AL: "HETEROGENEITY OF THERAPEUTIC RESPONSES IN ASTHMA" BRITISH MEDICAL BULLETIN, CHURCHILL LIVINGSTONE, LONDON, GB, vol. 56, no. 4, 2000, pages 1054-1070, XP001051229 ISSN: 0007-1420
- SAMPSON A P: "The leukotrienes: Mediators of chronic inflammation in asthma" CLINICAL AND EXPERIMENTAL ALLERGY, vol. 26, no. 9, 1996, pages 995-1004, XP001206742 ISSN: 0954-7894
- SAMPSON A P: "Eosinophils: Provokers or bystanders in asthma?" CLINICAL AND EXPERIMENTAL ALLERGY REVIEWS, vol. 1, no. 2, July 2001 (2001-07), pages 73-76, XP009048402 ISSN: 1472-9725
- BROUGHTON-KS: "Asthma and the role of dietary lipids" INTERNATIONAL SOCIETY FOR THE STUDY OF FATTY ACIDS AND LIPIDS NEWSLETTER, vol. 5, no. 1, 1998, XP002334662
- BROUGHTON K S ET AL: "REDUCED ASTHMA SYMPTOMS WITH N-3 FATTY ACID INGESTION ARE RELATED TO 5-SERIES LEUKOTRIENE PRODUCTION" AMERICAN JOURNAL OF CLINICAL NUTRITION, BETHESDA,MD, US, vol. 65, no. 4, 1997, pages 1011-1017, XP009048370 ISSN: 0002-9165
- SALVI SUNDEEP S ET AL: "The anti-inflammatory effects of leukotriene-modifying drugs and their use in asthma" CHEST, vol. 119, no. 5, May 2001 (2001-05), pages 1533-1546, XP002334663 ISSN: 0012-3692
- CHUNG K F ET AL: "Cytokines in asthma" THORAX, vol. 54, no. 9, September 1999 (1999-09), pages 825-857, XP002300990 ISSN: 0040-6376
- JONES T R ET AL: "Effects of a selective phosphodiesterase IV inhibitor (CDP-840) in a leukotriene-dependent non-human primate model of allergic asthma" CANADIAN JOURNAL OF PHYSIOLOGY AND PHARMACOLOGY, vol. 76, no. 2, February 1998 (1998-02), pages 210-217, XP009048397 ISSN: 0008-4212
- BACHERT CLAUS ET AL: "Total and specific IgE in nasal polyps is related to local eosinophilic inflammation" JOURNAL OF ALLERGY AND CLINICAL IMMUNOLOGY, vol. 107, no. 4, April 2001 (2001-04), pages 607-614, XP002334710 ISSN: 0091-6749
- ONO E. ET AL: 'Increase in Salivary Cysteinyl-Leukotriene Concentration in Patients with Aspirin-Intolerant Asthma.' ALLERGOL INT. [EPUB AHEAD OF PRINT] vol. 60, 24 November 2010,

## Description

### FIELD OF THE INVENTION

This invention relates generally to the diagnosis of asthma. More specifically, the invention provides kits and methods for differentiating those asthmatic patients who will respond to a particular type of treatment and those who will not, thereby streamlining therapeutic management of the disease.

### BACKGROUND OF THE INVENTION

Asthma may affect up to 5% of the Western population (Fleming et al., BMJ 294:279-283, 1987), and is the most common chronic condition of childhood with between 20 and 25% of all children experiencing wheezing at some point in their life. Although medical advances in the 19th and 20th centuries have established asthma as a diagnostic entity, asthma is a heterogeneous health problem that is difficult to classify (Kaliner et al., J. Am. Med. Assoc. 258:2851-2871, 1987*).* Recent statistics indicate that Wyoming is number one in the US for male pulmonary disease mortality (Amodio et al., Ladies Home Journal, pp 200, 1998)*.* Based on etiological classification, two types of asthma exist: extrinsic or atopic asthma, and intrinsic or cryptogenic asthma (Falliers et al., Ann Allergy, Vol. 53, pages 113-117, 1984). Current research has led to the characterization of asthma as a bronchial hyper-responsive chronic inflammatory disorder involving a variety of cells including mast cells, T lymphocytes (specifically Th₂ cells), macrophages, granulocytes, platelets, basophils and epithelial cells (Chanarin, et al., Drugs, Vol. 47, pages 12-24, 1994*;* Einarsson et al., Ann NY Acad Sci, Vol. 762, pages 89-100*).* Asthma can be characterized both clinically and pathologically. Clinically, asthma can be defined as a recurrent disease that causes intermittent wheezing, breathlessness, and sometimes a cough with sputum production. Pathologically, asthma characteristics include airflow obstruction due to a combination of smooth-muscle contraction, mucosal edema and inflammation, and viscid mucus secretion (Kaliner et al., J. Am. Med. Assoc. 258:2851-2871, 1987*).* While the disease involves both the large and small airways, the recognized pathophysiological events of asthma are a reduction in the small airway components (small bronchi and bronchioles) resulting in airway resistance, reduced forced expiratory volume and flow rates, and hyperinflation with trapping of lung air. Numerous biological compounds have been shown to be involved in both eliciting and promoting asthma.

For example, the asthmatic response can be precipitated by aspirin ingestion and exercise. Manifestation of the asthmatic response can be divided into three stages: a rapid spasmogenic phase, a late sustained phase and a subacute, chronic inflammatory phase (Holgate et al., Clin Allergy, Vol. 15, pages 221-234, 1985*;* Kay, Asthma: Clinical Pharmacology and Therapeutic Progress, pp 1-10, 1986). The immediate response is conventionally associated with pulmonary mast cell activation including the release of histamine and spasmogenic arachidonic acid metabolites of both the prostaglandin and leukotriene families. However, while histamine and the leukotrienes C₄,D₄, and E₄ seem to be key players in severe asthmatic reactions, they are not the exclusive mediators involved. Several cytokines appear to be involved in asthma either through their direct promotion or when lacking, their alleviation of symptomology.

Public health statistics from 1980 indicate that the prevalence of asthma in the US is 4.3 % or greater than 9.5 million individual cases. Further, prevalence is typically highest in children and the older adult population (Pendersen, et al., Allergy, Vol. 36, pages 175-181*;* Winder et al., Vital Heal th Stat, Vol. 10, pages 1-49, 1973). Difficulty in accurately diagnosing the prevalence of asthma can be attributed to the variety of methods used in diagnosis. Methods to examine prevalence include the use of questionnaires, telephone surveys, physical examinations, pulmonary function tests, allergen skin testing, and bronchial challenge tests (Bonner, Clin Chest Med, Vol. 5, pages 557-565, 1984). Accurate diagnosis is made more difficult by the fact that the asthma response and metabolites involved are not the same in all patients. Clearly, a need exists for an accurate diagnostic method for identifying those asthmatic patients who would benefit from administration of certain types of pharmacological agents.

### SUMMARY OF THE INVENTION

In accordance with the present invention, a method is provided for improved diagnosis and treatment of asthma patients based on altered cytokine and leukotriene profiles. The methods of the invention enable the clinician to accurately identify those asthmatic patients who are most likely to benefit from the different treatment protocols available for the management of the disease.

The invention provides a method as set out in claim 1 for differentially diagnosing asthma, to determine if a patient is a leukotriene based asthmatic or a cytokine based asthmatic. The invention further enables determining if a cytokine asthmatic is an IL-4 based, IL-16 based, or a GM-CSF based asthmatic.

A patient having symptoms of an asthma attack may be tested for cytokine and leukotriene levels. Both leukotrienes and cytokines can be measured using direct ELISA assays, or competitive binding assays. Any body fluid, including but not limited to blood, sputum, brochial lavage, and saliva may be tested. Alternatively, cells isolated from the patient may be assessed for the presence and levels of leukotrienes and cytokines. A pulmonary function test may have been administered to the patient, or a patient who presents with an asthmatic attack may be tested. The method may be performed immediately upon the onset of asthmatic symptoms, and may also include a test which is conducted after a suitable time period. For example, subsequent testing can be performed 4-6 hours post pulmonary function test or asthma attack.

An appropriate treatment protocol for the patient may be determined following diagnosis using the methods of the invention. Leukotriene based asthmatics will be treated with drugs that target leukotrienes, while cytokine based asthmatics will be administered an antagonist to their particular cytokine type. If no such antagonist is available, then traditional steroid therapy should be administered.

The invention also provides a kit for differentially diagnosing asthma. The kit includes, without limitation, agents which may be detectably labeled and which bind to leukotrienes C4, D4 et E4, and cytokines, including but not limited to IL-4, IL-16, and GM-CSF. Such molecules may be detected using immunoassays or hybridization/amplification assays. Accordingly, the kits of the invention may contain antibodies, detectable substrates or labels, polynucleotide probes or primers and vessels, containers or solid supports for performing such assays.

### BRIEF DESCRIPTION OF THE DRAWINGS

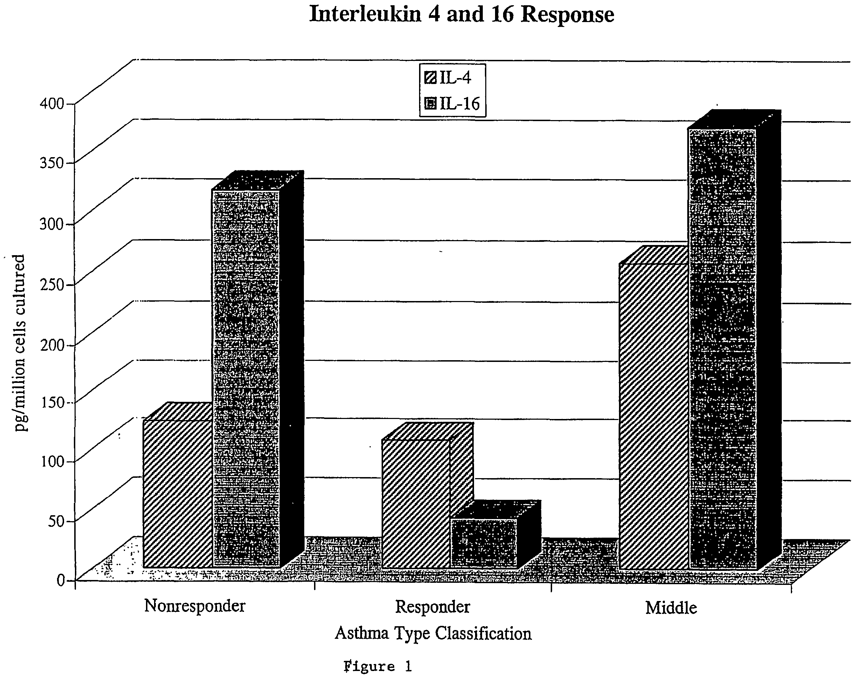

Figure 1 is a graph showing the classification of asthma patients based on interleukin 4 and interleukin 16 responses.

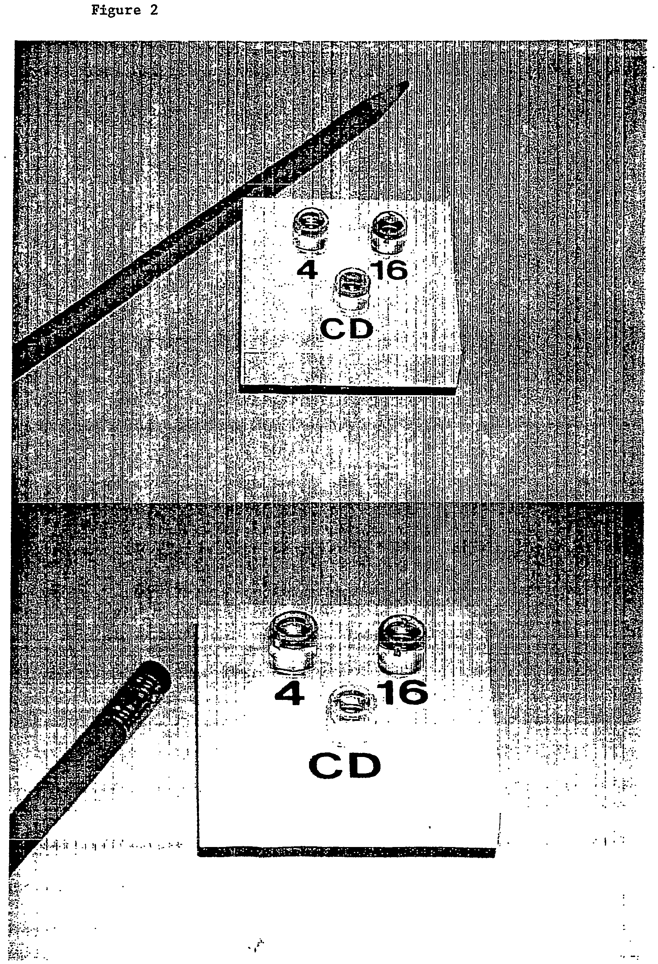

Figure 2 is a photograph of the platform used to assess leukotriene based asthmatics.
Figure 3 depicts the protocol for a typical pulmonary function test.

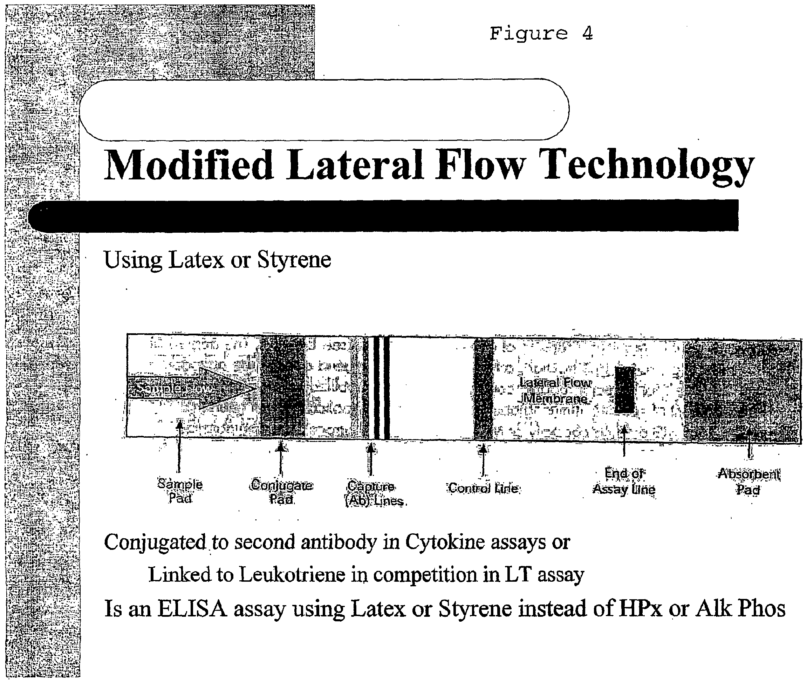

Figure 4 depicts a test strip which could be utilized to practice the instant invention.
Figure 5 is a table which depicts the leukotriene and cytokine profiles of various asthmatics after methacholine challenge.

### DETAILED DESCRIPTION OF THE INVENTION

The scope of the present invention is defined by the claims and any information that does not fall within the claims is provided for information only. Over ten million individuals are screened for the existence of asthma in the United States annually. Most treatment strategies rely on the assumption that asthmatics comprise a homogeneous population. In accordance with the present invention, it has been discovered that there are very distinct metabolic differences that exist in the asthmatic population. It has also been discovered that these metabolic differences are useful in guiding the clinician to the particular type of therapy. In preliminary studies, we have established that approximately one half of the asthmatic population may respond to dietary modification, thereby reducing the need for pharmacologic agents (Broughton et al., AJCN Vol. 65, pages 1011-1017*).* This *preliminary* study determined the effectiveness of (n-3) polyunsaturated fatty acid (PUFA) ingestion in ameliorating methacholine-induced respiratory distress in an asthmatic population. The ability of urinary leukotriene excretion to predict efficacy of (n-3) PUFA ingestion was assessed. Following ingestion of (n-3) PUFA at elevated levels, patient respiratory parameters were also assessed. Forced vital capacity (FVC), forced expiratory volume/second (FEV₁), peak expiratory flow (PEF), and forced expiratory flow 25%-75% (FEF₂₅₋₇₅) were measured along with weekly 24-Hour urinary leukotriene levels. Elevated (n-3) PUFA ingestion resulted in a positive methacholine bronchoprovocation dose change in over 40% of the test subjects (Responders). The provocative dose to cause a 20 percent reduction (PD₂₀) in FEV₁, FVC, PEF, and FEF₂₅₋₇₅ values became noncalculable due to a lack of significant respiratory reduction. Conversely, elevated (n-3) PUFA ingestion caused some of the patients (Nonresponders) to display further loss of respiratory capacity. Five-series leukotriene excretion with high (n-3) PUFA ingestion was significantly greater for responders than nonresponders. A urinary 4-series:5-series leukotriene ratio below 1, induced by (n-3) PUFA ingestion, may be predictive of respiratory benefit. As important as this finding was, it became equally as important to determine what differed in the nonresponding population and determine what inherent metabolic difference might exist between the different asthmatics.

Patients may have been administered a pulmonary function test according to standard protocols. Briefly, a methacholine challenge is administered, and cytokine and leukotriene levels in sputum immediately measured.

Patients may be administered various doses of methacholine. Saliva is recovered from patients and leukotrienes in the sample isolated and assayed. Asthmatics are then classified as leukotriene positive (responders) or negative (middle-responders or non-responders), depending on leukotriene levels at various methacholine doses. Patients with higher leukotriene levels were considered to be leukotriene based asthmatics, while patients with lower leukotriene levels were considered to be non-leukotriene, or cytokine based asthmatics. After a suitable time period (e.g., four, five, or six hours later), blood was drawn and plasma was tested for IL-4, IL-16, and GM-CSF levels. Based on plasma, saliva, and sputum profiles, it was determined if the cytokine based asthmatic patients were predominantly IL-4 based, IL-16 based, or GM-CSF based asthmatics.

Thus, methods of the invention enable the clinician to categorize asthmatics based on their profile of cytokine and leukotriene expression levels, and devise appropriate treatment protocols based on these profiles.

Specifically, differences in the expression levels of leukotrienes, interleukins, (IL-4, IL-16) and GM-CSF provide the means to differentiate between leukotriene based vs. specific cytokine based asthmatics. Currently the cost of asthma treatment can be in the excess of 200 dollars a month, and such treatment is ineffective in approximately half of the diagnosed cases. The method of the invention enables the clinician to identify the type of asthma-patient being assessed thereby identifying the ideal treatment method. Thus, practice of the method of the invention results in a significant financial savings to the patient and enables the clinician to select the correct pharmacological agent for treatment.

The following definitions are provided to facilitate an understanding of the present invention.

"Leukotrienes" are products of eicosanoid metabolism (usually arachidonic acid) with postulated physiological activity such as mediation of inflammation and allergic reactions. Leukotrienes differ from related prostaglandins and thromboxanes by not having a central ring. These molecules were designated leukotrienes because they were discovered in association with leukocytes and contain conjugated double bonds. Letters A through F identify the six metabolites thus far isolated with subscript numbers to indicate the number of double bonds bonds (e.g., leukotriene C₄).

"Cytokines" are hormone like low molecular weight proteins, secreted by many different cell types, which regulate the intensity and duration of immune responses, and are also involved in cell-to-cell communication. Exemplary cytokines include interferon, interleukins (such as IL-3, IL-4, IL-5, IL-8, IL-10, IL-13 and IL-16), GM-CSF, IFN-γ and lymphokines.

An "immune response" signifies any reaction produced by an antigen, such as a viral antigen, in a host having a functioning immune system. Immune responses may be either humoral in nature, that is, involve production of immunoglobulins or antibodies, or cellular in nature, involving various types of B and T lymphocytes, dendritic cells, macrophages, antigen presenting cells and the like, or both. Immune responses may also involve the production or elaboration of various effector molecules such as cytokines, lymphokines and the like. Immune responses may be measured both in *in vitro* and in various cellular or animal systems. Such immune responses may be important in protecting the host from disease and may be used prophylactically and therapeutically.

An "antibody" or "antibody molecule" is any immunoglobulin, including antibodies and fragments thereof, that binds to a specific antigen. The term includes polyclonal, monoclonal, chimeric, and bispecific antibodies. As used herein, antibody or antibody molecule contemplates both an intact immunoglobulin molecule and an immunologically active portion of an immunoglobulin molecule such as those portions known in the art as Fab, Fab', F(ab')2, F(v) and Sfv generated recombinantly.

With respect to antibodies, the term "immunologically specific" refers to antibodies that bind to one or more epitopes of a protein or compound of interest, but which do not substantially recognize and bind other molecules in a sample containing a mixed population of antigenic biological molecules.

A "direct binding assay" is generally known in the art, and is conducted in accordance with the procedures of Harlow and Lane, 1988. In general, a direct binding assay refers to an assay in which antiserum containing an anti-peptide antibody or ligand is added, and allowed to bind to a peptide. The binding of that antibody or ligand is then detected by a direct label, or by a secondary labeled antibody or ligand, which binds to the first antibody or ligand.

A "competitive binding assay" is generally known in the art, and is conducted in accordance with the procedures of Harlow and Lane, 1988. In general, a competitive binding assay refers to an assay in which unknowns are detected and quantified by their ability to block binding of a labeled known ligand to it's specific antibody.

The phrase "solid matrix" as used herein includes, without limitation, filter paper, multiwell dishes, microchips, derivatized magnetic particles and the like.

The term "tag," "tag sequence" or "protein tag" refers to a chemical moiety, either a nucleotide, oligonucleotide, polynucleotide or an amino acid, peptide or protein or other chemical, that when added to another sequence, provides additional utility or confers useful properties, particularly in the detection or isolation, of that sequence or protein. Thus, for example, a homopolymer nucleic acid sequence or a nucleic acid sequence complementary to a capture oligonucleotide may be added to a primer or probe sequence to facilitate the subsequent isolation of an extension product or hybridized product. In the case of protein tags, histidine residues (e.g., 4 to 8 consecutive histidine residues) may be added to either the amino- or carboxy-terminus of a protein to facilitate protein isolation by chelating metal chromatography. Alternatively, amino acid sequences, peptides, proteins or fusion partners representing epitopes or binding determinants reactive with specific antibody molecules or other molecules (e.g., flag epitope, c-myc epitope, transmembrane epitope of the influenza A virus hemaglutinin protein, protein A, cellulose binding domain, calmodulin binding protein, maltose binding protein, chitin binding domain, glutathione S-transferase, and the like) may be added to proteins to facilitate protein isolation by procedures such as affinity or immunoaffinity chromatography. Chemical tag moieties include such molecules as biotin, which may be added to either nucleic acids or proteins and facilitates isolation or detection by interaction with avidin reagents, and the like. Numerous other tag moieties are known to, and can be envisioned by the trained artisan, and are contemplated to be within the scope of this definition.

"N-3 polyunsaturated fatty acids" are found in fish and upon ingestion have been found to ameliorate methacholine-induced respiratory distress in a certain portion of the asthmatic population.

A "sample" or "patient sample" or "biological sample" generally refers to a sample which may be tested for a particular leukotriene or cytokine profile. Samples may include but are not limited to blood, serum, plasma, urine, saliva, sputum, bronchial lavage and the like. Most preferably, the sample is a saliva, sputum, blood, and/or plasma sample.

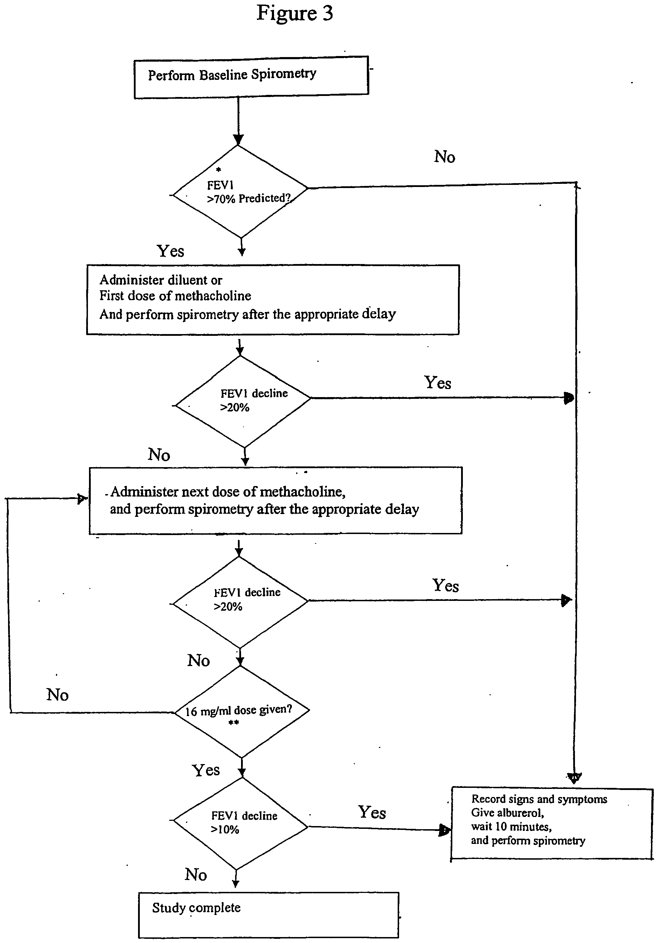

A "Pulmonary Function Test" is a test which measures the amount of air that is taken into the lungs and how quickly it can be expelled. Often, a pulmonary function test is conducted to determine the effects of various conditions and compounds on pulmonary function. For example, a pulmonary function test can be used to assess and monitor the nature of an asthmatic attack, as well as to measure the potential therapeutic effect of an agent on an asthma attack. Guidelines for such tests are standard in the art, and may be found for example, in The American Journal of Respiratory and Critical Care Medicine Vol 161:309-329, 2000, and are illustrated in Figure 3. Briefly, an agent which alters pulmonary function is administered, and breathing is monitored for an appropriate period of time. Such agents include without limitation, methacholine, Histamine, Manitol, Adenosine, FELD (Cat Dander), Dust Mite Allergen, and Specific Allergens.

A "methacholine challenge" is a type of pulmonary function test in which methacholine is administered and pulmonary function is evaluated. A "methacholine challenge" as described herein may be a "traditional methacholine challenge" or a "progressive methacholine challenge". Typically a traditional methacholine challenge will use a protocol similar to that set forth in Figure 3. Normal concentrations and cumulative doses of methacholine used in a traditional methacholine challenge can be as follows: 0.025 mg/ml (0.125 units), 0.25 mg/ml (1.375 units), 2.5 mg/ml (13.875 units) and 10 mg/ml (63.875 units), where units are equal to milligrams of methacholine. Typically, a progressive methacholine challenge uses lower doses of methacholine. The following concentrations and cumulative doses of methacholine would be considered a progressive methacholine challenge: 0.025 mg/ml (0.125 units), 0.25 mg/ml (1.375 units), 0.5 mg/ml (3.875 units), 1 mg/ml (8.875 units), 2 mg/ml (18.875 units), 2.5 mg/ml (31.375 units) and 10 mg/ml (81.375 units), and 25 mg/ml (206.375 units). A progressive methacholine challenge can be a single dose of methacholine. In this single dose challenge, a total methacholine dose of 15 mg/ml or less, or 8 mg/ml or less is administered.

### I. Preparation of Leukotriene and Interleukin specific antibodies

The present invention may make use of antibodies capable of immunospecifically binding to leukotrienes and cytokines including leukotriene C₄, D₄, and E₄ and interleukins 4 and 16, and Granulocyte Macrophage Colony Stimulating Factor. Antibodies for detecting the molecules set forth above have been prepared according to general methods following standard protocols and may be may be purchased commercially from R & D Systems, Minneapolis, MN; and Assay Designs, Inc., Ann Arbor, MI.

Polyclonal or monoclonal antibodies immunologically specific for cytokines, including interleukin proteins or leukotrienes may be used in a variety of assays designed to detect and quantitate the molecules for the purposes of assessing the asthmatic patient. Such assays include, but are not limited to: (1) flow cytometric analysis; (2) immunochemical detection/localization of cytokines and/or leukotrienes in patient body fluids, including saliva, sputum, blood, bronchial lavage, and plasma; and (3) immunoblot analysis (e.g., dot blot, Western blot) of extracts from various cells. Additionally, anti-cytokine or anti-leukotriene antibodies can be used for purification of cytokine and interleukin protein and any associated subunits (e.g., affinity column purification, immunoprecipitation).

### II. Kits for Performing the Disclosed Method

Kits are also provided to facilitate the detection of the leukotrienes and cytokines IL-4, IL-16, *GM-CSF,* and leukotriene C₄, D₄, and E₄ in biological samples.

In certain embodiments, the present invention makes use of immunodetection methods for binding, purifying, removing, quantifying or otherwise generally detecting biological components. In general, the immunobinding methods include obtaining a sample suspected of containing a protein or peptide, and contacting the sample with an antibody in accordance with the present invention, as the case may be, under conditions effective to allow the formation of immunocomplexes.

The immunobinding methods include methods for detecting or quantifying the amount of a reactive component in a sample, which methods require the detection or quantitation of any immune complexes formed during the binding process.

In a simultaneous analysis of IL-4, IL-16, *GM-CSF,* and leukotriene C₄/D₄/E₄ from patient saliva, if the patient is leukotriene C₄, D₄, or E₄ positive, a leukotriene based asthmatic has been identified. Thus, drugs such as Accolate (zafirlukast), Sigulair (montelukast) and Zyflo (zileuton) which are C₄/D₄ receptor antagonists, or, a 5 lipoxygenase inhibitor and dietary n-3 fatty acids would benefit such patients. If elevated IL-16, elevated IL4, and low leukotriene C₄ expression levels are identified, such patients are more likely to benefit from the administration of certain new IL-4 receptor antagonists with essentially no benefit being obtained from administration of the drugs set forth above for leukotriene based asthmatics. Such patients are referred to as middle responders. Finally, if elevated IL-16, moderate IL-4 and low leukotriene C₄ levels are observed, such patients are classified as non-responders. These asthmatics are treated with IL-16 or IL-4 receptor antagonists, and will also most likely benefit from standard steroid therapy.

In terms of antigen detection, the biological sample analyzed may be any sample that is suspected of containing the cytokines (including interleukins), or leukotriene. Suitable samples include saliva, sputum, bronchial lavage, an isolated cell, a cell membrane preparation, separated or purified forms of any of the above protein-containing compositions, or even any biological fluid that comes into contact with airway tissues, including blood and lymphatic fluid.

Contacting the chosen biological sample with an antibody under conditions effective and for a period of time sufficient to allow the formation of immune complexes (primary immune complexes) is generally a matter of simply adding the composition to the sample and incubating the mixture for a period of time long enough for the antibodies to form immune complexes with, i.e., to bind to, any antigens present. After this time, the sample-antibody composition, such as a tissue section, ELISA plate, dot blot or Western blot, will generally be washed to remove any non-specifically bound antibody species, allowing only those antibodies specifically bound within the primary immune complexes to be detected.

In general, the detection of immunocomplex formation is well known in the art and may be achieved through the application of numerous approaches. These methods are generally based upon the detection of a label or marker, such as any radioactive, fluorescent, biological or enzymatic tags or labels of standard use in the art. U.S. Patents concerning the use of such labels include U.S. Pat. Nos. 3,817,837; 3,850,752; 3,939,350; 3, 996, 345; 4,277,437; 4,275,149 and 4,366,241. Of course, one may find additional advantages through the use of a secondary binding ligand such as a second antibody. The second antibody may optionally be color labeled, or latex labeled. Alternatively, a biotin/avidin ligand binding arrangement may be utilized. All of these labeling and detection methods are known in the art.

The immunodetection methods of the present invention have evident utility in the diagnosis and characterization of asthma. Here, a biological or clinical sample suspected of containing either the cytokine (including interleukin) or leukotriene is used.

In one broad aspect, the present invention encompasses kits for use in detecting expression levels of cytokines including interleukins and leukotrienes in biological samples. Such a kit may comprise one or more pairs of primers for amplifying nucleic acids corresponding to the IL-4, IL-16, and GM-CSF genes. The kit may also comprise buffers, nucleotide bases, and other compositions to be used in hybridization and/or amplification reactions. Each solution or composition may be contained in a vial or bottle and all vials held in close confinement in a box for commercial sale. Another embodiment of the present invention encompasses a kit for use in detecting IL-4, IL-16, GM-CSF and leukotriene antigens in biological specimens. Such a kit may comprise antibodies or antibody fragments immunologically specific for IL-4, IL-16, GM-CSF and leukotriene and means for assessing the formation of immunocomplexes containing these molecules.

### EXAMPLE I

The following materials and methods are provided to facilitate the practice of Example I.

### Subjects

Thirty-four, non-smoking atopic asthmatic subjects with nonspecific bronchial responsiveness to methacholine with a forced expiratory volume in 1 s (FEV₁) above 70% predicted, and seventeen, nonsmoking nonasthmatic healthy control subjects were recruited from the Laramie, WY community. Subjects were screened for overall health status, use of drugs which affect blood pressure, asthma or eicosanoid synthesis, and fish (or fish oil) consumption. Potential subjects who ingested fish oil supplements at any level, or whose pattern of fish consumption was greater than one meal/week were not selected. Individuals with bleeding disorders or a history of delayed clotting time were not considered for this study. Asthmatic subjects were on various treatments including inhalants such as salbutamol, steroids, and oral ingestion of theophyllines. No subject had an upper respiratory tract infection or exacerbation of asthma in the previous six weeks prior to study initiation. Nonsteroidal anti-inflammatory drugs were not allowed during the study. The study was approved by the Human Subjects Review Board at the University of Wyoming, and Informed Consent was obtained from all participants following careful description of the study design and expectations. Twenty-nine asthmatics and fifteen controls completed the study.

Seven-day food records for all participants were examined to determine normal (n-6) PUFA consumption. One random three-day diet analyses were conducted throughout the study to monitor for changes in diet patterns and (n-6) PUFA intake. Subjects participated in a one month study examining the effect of (n-3) PUFA at a (n-3):(n-6) ratio of two on respiratory parameters, urinary metabolites, and immune cell product profiles. The treatment consisted of a one month supplementation period with a (n-3):(n-6) PUFA ratio of 1:2. The fish oil regimen was individualized for each participant and altered if necessary throughout the study, based on the random three day food record analyses. Encapsulated fish oil was generously donated by the Shaklee Corporation (Hayward, CA). Subjects were given the appropriate number of oil capsules divided into daily allotments at the beginning of each study week.

Two 24-hour urine samples were collected in opaque bottles on the two days immediately preceding the study to serve as baseline samples. Samples were acidified with formic acid to a final concentration of 3 mM and diluted with methanol to a final concentration of 10 %. FVC, FEV₁ as an assessment of large airway capacity, PEF as an assessment of medium airway capacity, and FEF₂₅₋₇₅ values as an assessment of small airways were obtained immediately preceding the study to serve as baseline values. 24 hour urine specimens were obtained the last day of each week during the 4 week oil supplementation period to monitor potential progressive changes in eicosanoid metabolism and to monitor diet compliance. Following determination of baseline and treatment urine volumes, LTs were extracted from a 200 ml aliquot and frozen at -80 °C for subsequent analysis.

### Study Protocol

At baseline and following the four week treatment period, patients entered the participating physician's office for assessment of respiratory status via determination of FVC, FEV₁, FEF25-75, and PEF following a methacholine (Provocholine, Roche Laboratories, Nutley, NJ) challenge. Methacholine was administered sequentially through five inhalations in serial concentrations with a Salter Lab Series 8900 nebulizer (Salter Labs, Arvin, CA) at a flow rate of 7-8 L/min, such that the total administered was 0, 0.125, 1.375, 13.88, and 63.88 cumulative units. All respiratory parameters were determined within five minutes. The procedure was terminated if there was a 20% or greater reduction in FEV₁ compared to a baseline saline (0.9% NaCl with 0.4% phenol pH 7.0) solution or when 63.88 cumulative units had been administered. If there was a reduction of 15% to 19% in FEV₁, the challenge was repeated at that concentration or the next higher concentration as long as the cumulative units did not exceed 63.88. Methacholine challenge FVC, FEV₁, FEF₂₅₋₇₅, and PEF values were obtained by the attending physician through the use of a Brentwood 2000 spirometer (Brentwood). Sputum was collected at the time of methacholine challenge from one half of the subjects and stored at -70 ° C until analysis.

### Leukotriene Analysis

Leukotrienes were extracted from 200 ml of freshly collected acidified urine and from the 2 ml of cell culture media described following. 100 ng of prostaglandin B₁ (PGB₁) was added to the 200 ml aliquot for use as an internal standard. Leukotrienes were isolated by solid phase extraction over C18 cartridges (Supelclean LC-18, Supelco, Inc., Bellefonte, PA) that were prewashed sequentially with 10 ml methanol, 5 ml water, and 5 ml hexane. After application of sample to the cartridge, cartridges were washed sequentially with 10 ml methanol/water (1:9, v/v), 10 ml water, and 5 ml hexane followed by elution of LTs with 2 ml of methanol. Following elution, LT extractions were stored in methanol at -80 °C for subsequent analysis. At analysis, samples were evaporated to dryness under nitrogen with the resulting residue reconstituted in the HPLC solvent system of methanol:water (65:35, v/v), pH 4.68, containing 5 mM ammonium acetate and 1 mM EDTA. All samples were analyzed in duplicate with averages being used for statistical analysis. The leukotrienes were separated by reverse phase high pressure liquid chromatography (RP-HPLC) on a Partisphere C-18 column (6mm x 12.5 cm, Whatman, Hillsboro, OR) with a flow rate of 1.0 ml/min and quantified using a Hewlett-Packard 10409A Diode Array spectrophotometer (Hewlett-Packard, Liverpool, NY) by monitoring at 280 nm. All leukotrienes were identified by their distinctive UV absorption spectra, and comparison of retention times with known standards. Leukotrienes were quantified against the internal PGB₁ standard using extinction coefficients of authentic standards (Orning, et al., Eur. J. Biochem, Vol. 120, pages 44-45, 1981*)* Leukotrienes E₄, E₅, and N-acetyl LTE₄ (N-ac LTE₄) were purchased from Caymen Chemical (Ann Arbor, MI).

### Cell culture

At the inception of the study and following the (n-3) fatty acid feeding period, 21 ml venous whole blood was isolated from each subject over heparin as an anticoagulant. Immune cells (primarily peripheral monocytes) were isolated over a ficol gradient and cultured at 2x10⁶ cells per ml in RPMI plus 10% fetal bovine serum (FBS) buffer. Cells were stimulated with phytohemaglutinin (PHA) or lipopolysaccharide (LPS) for 48 hours to induce cytokine production, or A23187 for 4 hours to stimulate leukotriene synthesis. Leukotrienes were analyzed as cited above. The cytokines, INF-γ, IL-3, IL-4, IL-5, IL-8, IL-10, IL-13, and IL-16 were analyzed by ELISA assay with the kits purchased from the following companies: INF-γ, IL-10, and IL-13 were purchased from Research Diagnostics, Inc., Flanders, NJ; IL-4 and IL-5 were purchased from Assay Designs, Inc. Ann Arbor, MI; and IL-3, IL-8, and IL-16 were purchased from Biosource International Camarillo, CA. The ELISA assays were sandwich assays. An initial antibody to the cytokine being examined is linked to a microtiter plate by incubation. The samples are then added to the wells and incubated to allow formation of the antigen-antibody complex. The well is then triple washed and incubated with a second antibody with a horseradish peroxidase conjugate. Following another triple wash, the well is then incubated with hydrogen peroxide and TMB or another substrate for color development. Color positive in this assay is then used to assess the presence of cytokine and quantitate the level of biosynthesis based on the degree of color development.

### Statistics

The number of replicates for each assay was determined by statistical analyses of the power to detect a physiologically significant difference between treatments. Estimates of variance used in the design are computed from previous experiments. As an example, urinary leukotriene levels were judged to be importantly different if a dietary-induced 15% decrease occurred. Differences between means for total LT excreted, and diminishment at each methacholine dose for FVC, FEV₁, FEF25-75, and PEF and the PD₂₀ for each of these parameters were assessed by ANOVA. All statistical analyses were conducted with SAS (Statistical Analysis Systems Institute, Inc. Cary, NC). When overall differences were detected, specific treatment differences were assessed using Duncan's protected least significant difference test. Significance was determined at P<0.05. Values are expressed in the text as mean ±SEM with a common n=17 for responder cases, n=12 in nonresponder cases, and n=15 for nonasthmatic controls unless stated otherwise.

### Results

### Pulmonary Function Tests During Methacholine Bronchoprovocation

With (n-3) PUFA ingestion at a (n-3) to (n-6) PUFA ratio of 1:2, respiratory parameters in the total sample population were essentially unchanged compared to baseline responses. Upon examination of these data, it became evident that breathing capacity was actually improved in 17 of the completing participants (Responders) with no change in the 12 nonresponding asthmatics. When further examining the pulmonary results, it became evident that variations in the responder group consisted of 15 individuals with marked respiratory improvement and two individuals with only a minor improvement in respiratory function with (n-3) PUFA ingestion. Further, when the nonresponders were scrutinized more closely, there were 5 nonresponders that did not display the same degree of respiratory difficulty as that seen by the other 7 nonresponders, and were close to the average seen by all the asthmatics when averaged together. These latter 5 nonresponders and the preceding 2 responders that did not achieve complete respiratory benefit were lumped into what now is called the "MIDDLE GROUP". When data from the responders are summarized separately, there is virtually no diminishment in any respiratory parameter with increasing methacholine challenge even at 63.88 units of methacholine. The middle group was able to achieve a methacholine cumulative dose of 13.88 units while the nonresponders, as a group, were generally unable to continue beyond a cumulative dose of 13.88 units of methacholine, and demonstrated enhanced breathing difficulty at 1.375 units methacholine that was exacerbated when methacholine was increased to 13.88 units.

### Leukotriene Quantitation

Compared to baseline, average urinary 4-series LT excretion in responders decreased while LTE₅ excretion increased in response to (n-3) PUFA ingestion. Conversely, urinary 4-series LT excretion in nonresponders was only mildly reduced while LTE₅ excretion increased significantly. Sputum LT were evident although demonstrating a markedly different pattern of synthesis when comparing the responders and nonresponders.

### Cytokine Profiles

Cytokine profiles differed significantly in some cases and showed very little difference in other cases. In particular, in cultured immune cells in response to (n-3) PUFA ingestion, there was either no change or a mild increase in IL-3, IL-8, IL-10, IL-13, in the asthmatic responders and the middle group with a mild decrease in the nonresponding participants in response to PHA cell stimulation. In response to LPS stimulation, IL-3 was unchanged in the nonresponder population yet showed a marked increase in response to (n-3) PUFA ingestion in the responder population. Similarly, IL-5 demonstrated a sharp increase in production in the responders following (n-3) PUFA ingestion following PHA stimulation. While IL-10 remained unchanged or increased following (n-3) PUFA ingestion in response to PHA stimulation, with LPS stimulation there was a significant reduction in IL-10 production in all three groups. INF-γ was reduced in the nonresponders in response to (n-3) PUFA ingestion when cells were stimulated with either PHA or LPS. Conversely, INF-γ increased in both the responders and what is being termed the middle group following (n-3) PUFA ingestion when cells were stimulated with either PHA or LPS. While IL-4 synthesis was reduced in all groups with (n-3) PUFA ingestion, IL-16 was reduced in the nonresponders and increased in the responders following LPS or PHA stimulation: In the middle group, IL-16 was reduced 9-fold following (n-3) PUFA ingestion in LPS stimulated cells but remained virtually unchanged in the PHA stimulated cells.

More significant than the response to (n-3) PUFA ingestion was the difference in production of different cytokines following PHA or LPS cell stimulation. While INF-γ, IL-3, IL-8, IL-10, and IL-13 did vary with (n-3) PUFA ingestion and differed considerably from nonasthmatic controls in some cases, the differences in these metabolites between the responding and nonresponding asthmatics at baseline and with (n-3) PUFA ingestion did not vary significantly. When these cytokines did vary, it was in response to (n-3) PUFA ingestion as was the case for IL-3 with LPS stimulation or IL-10 with PHA stimulation. However, unlike these five cytokines, IL-4, -5 and -16 varied considerably at both baseline testing and with (n-3) PUFA ingestion. More significantly, IL-4 was 1.25-fold higher in the nonresponding asthmatics at baseline when compared with the responders but was virtually the same as the responders following (n-3) PUFA ingestion. However, the middle group demonstrated a 2.2-fold higher level of IL-4 than the responders at baseline that increased to 2.7-fold higher with (n-3) PUFA ingestion. IL-5 was 1.3-fold higher in the responder asthmatics at baseline and increased to 2.2-fold that seen in the nonresponding asthmatics following (n-3) PUFA ingestion. The middle group demonstrated an even greater difference when compared with the nonresponders and was 2-fold higher at baseline and 2.3-fold higher with (n-3) PUFA ingestion. IL-16 synthesis in response to cell stimulation was 7.4-fold higher in the nonresponding and 8.6-fold higher in the middle asthmatics when compared to the responding asthmatics at baseline. When (n-3) PUFA were consumed, IL-16 release was significantly reduced in both the middle group and the nonresponders and was increased in the responders to the point that IL-16 release in the responders was 3.3-fold and 1.4-fold higher than that seen in the middle group and the nonresponders, respectively.

The sputum of the asthmatics showed this same degree of variability with even larger differences in IL-4 and IL-16 production. The sputum of the nonresponding asthmatics contained 6.4- and 11-fold more IL-16 and IL-4, respectively, than that seen in the responding asthmatics.

### Discussion

Ingestion of the (n-3) polyunsaturated fatty acids (PUFA) found in fish oil can markedly alter the pattern of LT production in an individual with these differences having particular relevance in the amelioration of respiratory difficulty in certain types of asthmatics.

It is now recognized that (n-3) PUFA may be a significant dietary means to help control asthma. The sulfidopeptide leukotrienes (SP-LT) have been implicated in inflammatory conditions of the skin (psoriasis) (*Brain, et al., J.*

*Invest. Dermatol., Vol. 83, pages 70-73, 1984*), the lung (allergic asthma) (Chanarin, Drugs, Vol. 47, pages 12-24, 1994*),* joints (rheumatoid arthritis) (Kremer et al., Arth. Rheum, Vol. 33, pages 810-820, 1990*)* and the heart (myocardial infarction) (Brain et al., Pharmacol. Ther., Vol. 46, pages 57-66, 1990*).* Recent recommendations to increase consumption of fish or fish products are motivated by research which demonstrated that when (n-3) PUFA are present at a dietary ratio of between 1:5 and 1:2.5 with dietary (n-6) PUFA (found in vegetable oil), there is a reduction in overall eicosanoid production which may reduce risk of a host of pathophysiologies.

In a previous study, we found that dietary supplementation with fish oil at a (n-3) to (n-6) PUFA ratio 1:2, alleviated asthma symptoms in over 40% of those tested. This study confirms these findings and indicates that the benefit achieved with (n-3) PUFA ingestion may be present in closer to 50% of the asthmatic population. Further, the average effective dose of fish oil provided approximately 3.3 g of EPA and DHA daily.

Previously, it was reported that consumption of 1 g/day of DHA and EPA for one year beneficially altered FEV₁ values in allergic asthmatic patients (Dry et al., Int Arch Allergy Appl Immunol, Vol. 95, pages 156-157, 1991*).* A similar study utilizing 5.4 g of EPA and DHA daily for 10 weeks did not demonstrate any effect in the total subject population following histamine challenge. However, 6 of the 11 subjects exhibited minor respiratory improvements. In a subsequent study (Arm et al., Am Rev Respir Dis, Vol. 139, pages 1395-1400, 1989*),* subjects who consumed (n-3) PUFA as max-EPA (an omega-3 product) did not exhibit respiratory improvements immediately following histamine challenge, but showed significant improvements in their recovery periods. These findings when considered in conjunction with the present data indicate that (n-3) PUFAs, when consumed long enough, or at high enough levels, may be of benefit in the amelioration of asthmatic parameters in a portion of the asthmatic population.

The cytokine data demonstrated that there may be significant changes in profiles following cell stimulation in response to (n-3) PUFA ingestion in some cases, i.e. IL-3 and IL-10, and very little change in others, i.e. IL-8, IL-13, and INF-γ. However, significant basal differences exist in IL-4, -5, and -16 production between nonresponding, middle and responding asthmatics with the nonresponders demonstrating significantly higher levels of both IL-4 and -16 and lower levels of IL-5 than that seen in responders. Conversely, the middle asthmatics have significantly higher levels of IL-4, -5, and -16 than the responders when not consuming (n-3) PUFA with similar IL-5 levels and lower IL-16 levels following (n-3) PUFA ingestion. Responding asthmatics appear to rely significantly on leukotrienes to drive their asthmatic response and while the nonresponding and middle asthmatics showed some IL-16 associated potential benefit with (n-3) PUFA ingestion, and do not appear to rely as heavily on leukotrienes in their asthmatic response. This finding may partially explain why LT receptor antagonists, and agents that block LT biosynthesis only appear to be beneficial in approximately one-half of asthmatics, the leukotriene based asthmatics. The other half of the asthmatic population may be more of a cytokine type of asthma that based on these results may be driven through elevated production of either IL-4 or IL-16, or both. Based on these results and the presence of these proasthmatic cytokines and leukotrienes in the sputum of the asthmatic, it is possible to use the differences that exist in this fluid or another body fluid to differentiate between the types of asthma and design type specific methods of treatment. Thus, the method of treatment could include administration of agents such as zafirlukast and montelukast that block LTC₄, LTD₄ and LTE₄ binding or zileuton, an inhibitor of the 5 lipoxygenase responsible for LT synthesis for LT based asthmatics. An IL-4 or IL-16 receptor antagonist can be administered for the treatment of cytokine based asthmatics. To date there is an IL-4 antagonist in testing while an IL-16 antagonist has not been identified in the literature. In the latter case, until an IL-16 antagonist is developed, these patients are treated with standard steroid therapy.

The beneficial change in respiratory parameters documented in responders is likely primarily attributable to an overall increase in 5-series SP-LT production. A necessary shift in LT biosynthesis in which 5-series LT increase is coupled with reductions in 4-series LT synthesis to the point where the 5:4 ratio exceeds one seems to be critical for mediating an improved response to methacholine challenge. Furthermore, the ineffectiveness of (n-3) PUFA ingestion in nonresponders appears to be mediated more through a lack of increased 5-series LT production than the reduction in 4-series LT synthesis. The presence of significant differences in both the LT and cytokine profiles between different types of asthmatics is both novel and significant in explaining differences that exist between responding and nonresponding asthmatics. As the 5-series LT have been shown to be less biologically potent in the guinea pig ileum (Hammarstrom, J. Biol. Chem, Vol. 255, pages 7093-7094, 1980*)* and eicosapentaenoic acid has been shown to inhibit the release of anaphylactic cyclooxygenase products in guinea-pig lung parenchymal strips while enhancing SP-LT release (Simmet et al., Arch. Pharm, Vol. 335, pages 652-659, 1987*),* the respiratory benefit associated with (n-3) PUFA ingestion in the responders could be associated with the inability of 5-series LT to elicit an asthmatic response, or by competitive inhibition by 5-series LT at the 4-series LT receptors. In the nonresponders, the lack of an effect of (n-3) PUFA ingestion might be attributable to the presence of a cytokine based asthmatic response versus a leukotriene driven asthmatic response.

In summary, pharmacologic intervention may not be necessary for leukotriene based asthmatics if they are able to incorporate a source of (n-3) PUFA in their diet, i.e. fish or fish oil, to alleviate minor respiratory problems. However, if this cannot be accomplished, this family of asthmatics may need to rely on agents that impede leukotriene synthesis or block leukotriene binding. For the other asthmatics, new IL-4 therapy or continued steroid therapy may be their only recourse in the treatment of asthma until specific treatment agents are developed.

### Example 2

To confirm that different types of asthmatics exhibit various cytokine and leukotriene profiles, and to further categorize the subcategories of cytokine based asthmatics the following experiments were performed.

### Materials and Methods:

First, a traditional methacholine challenge was administered. Normal concentrations and cumulative doses of methacholine, in parentheses, used in the pulmonary function test were as follows: 0.025 mg/ml (0.125 units), 0.25 mg/ml (1.375 units), 2.5 mg/ml (13.875 units) and 10 mg/ml (63.875 units), where units are equal to milligrams of methacholine. Immediately following methacholine challenge, sputum was collected from the subjects, and assayed for leukotriene, IL-4, and IL-16 levels.

### Results:

Profiles were evaluated by cytokine levels as set forth above. The results of these experiments and the subsequent determination of asthmatic type are summarized in Table I:

**Table I:**

| Leukotriene and Cytokine Profiles in the Sputum of Patients in Response to Traditional Methacholine Challenge | | | | | | | |
|---|---|---|---|---|---|---|---|
| | | | | Cytokines | | | |
| # | Type | | Subject | IL-16 | IL-4 | | Leukotriene |
| | | | | | | | |
| 1 | N | | 1-1 | 359.500 | 44.5 | | 137.3 |
| 2 | N | | 18-1 | 291.667 | 39 | | 223.0 |
| 3 | N | | 33-1 | 280.500 | 35.75 | | 85.5 |
| 4 | N | | 52-1 | 281.000 | 47.1 | | 37.9 |
| 5 | N | | 53-1 | 357.500 | 9.6 | | 16.7 |
| 6 | N | | 55-1 | 361.667 | 113.1 | | 729.0 |
| 7 | N | | 56-1 | 302.000 | 1.8 | | 173.8 |
| | | | | | | | |
| | | | average | 319.119 | 41.550 | | 200.439 |
| | | | *average | | | | 112.346 |
| | | | | | | | |
| 8 | Nm | | 24-1 | 1006.667 | | | 151.6 |
| 9 | Nm | | 47-1 | 931.667 | 9.6 | | 202.3 |
| 10 | Nm | | 49-1 | 906.667 | 29.4 | | 93.6 |
| 11 | Nm | | 51-1 | 611.667 | 128.75 | | 16.5 |
| 12 | Nm | | 54-1 | 1220.000 | 21.9 | | 0.0 |
| | | | | | | | |
| | | | average | 935.333 | 47.413 | | 92.790 |
| | | | | | | | |
| | | | | | | | |
| 13 | R | | 13-1 | 32.000 | 1.2333 | | 272.7 |
| 14 | R | | 15-1 | 30.750 | 0 | | 208.6 |
| 15 | R | | 20-1 | 95.833 | 4.8 | | 260.1 |
| 16 | R | | 21-1 | 130.000 | 6.2 | | 564.3 |
| 17 | R | | 32-1 | 119.167 | 2.75 | | 302.6 |
| 18 | R | | 34-1 | 89.000 | 1.9 | | 115.7 |
| 19 | R | | 44-1 | 173.000 | 0 | | 498.6 |
| 20 | R | | 46-1 | 121.000 | 0 | | 265.1 |
| 21 | R | | 48-1 | 83.000 | 20.7 | | 486.1 |
| 22 | R | | 50-1 | 72.500 | 0 | | 232.4 |
| | | | | | | | |
| | | | average | 94.625 | 3.758 | | 320.603 |

| | | | | | | | |
|---|---|---|---|---|---|---|---|
| N: Non-Responder; Nm: Middle Responder; R: Responder | | | | | | | |

Patients who exhibit high leukotriene levels in response to the traditional methacholine challenge are designated leukotriene based asthmatics. When a low or lack of production of leukotriene in sputum was observed in response to methacholine challenge, asthmatics were identified as cytokine based asthmatics. Plasma IL-4 and IL-16 profiles were used to identify subjects as either a predominantly a IL-4 based asthmatic, a IL-16 based asthmatic.

### Discussion:

The results of these experiments indicate that it is possible to differentiate leukotriene based asthmatics from cytokine based asthmatics with the cytokine based asthmatics being subdivided into different subgroups. Such determination provides valuable information for specific targeted asthma therapy.

### Example III

Two objectives were achieved in the present example. First, a progressive methacholine challenge protocol has been developed. This protocol utilizes the minimal level of methacholine at which an asthmatic reaction is elicited in a pulmonary function test. Second, different body fluids were tested at different times for cytokine and leukotriene levels to determine which body fluid and assay protocol were most effective for asthma subtype determination.

First, tests were conducted to determine the minimal level of methacholine necessary to differentiate between these different asthma subtypes. Various doses of methacholine were administered and the minimal dose which induces an asthma attack producing measurable levels of leukotrienes and/or cytokines, (e.g., interleukin-4 (IL-4), IL-16, or granulocyte macrophage-colony stimulating factor (GM-CSF)) was determined. Various body fluid samples were taken immediately, and 4-6 hours following methacholine challenge. These samples were assayed for leukotriene and cytokine levels as described below.

### Materials and Methods:

### Methacholine Tests

Normal concentrations and cumulative doses of methacholine, in parentheses, typically used in a pulmonary function test are as follows: 0.025 mg/ml (0.125 units), 0.25 mg/ml (1.375 units), 2.5 mg/ml (13.875 units) and 10 mg/ml (63.875 units), where units are equal to milligrams of methacholine, and examined a more progressive dose of methacholine. A lower, more progressive dose of methacholine was examined in the instant protocol, as many asthmatics will typically have an asthmatic reaction in the lower cumulative dose range. In this revised test the following concentrations and cumulative doses of methacholine were examined: 0.025 mg/ml (0.125 units), 0.25 mg/ml (1.375 units), 0.5 mg/ml (3.875 units), 1 mg/ml (8.875 units), 2 mg/ml (18.875 units), 2.5 mg/ml (31.375 units) and 10 mg/ml (81.375 units), and 25 mg/ml (206.375 units).

### Leukotriene and Cytokine Assays

Following each methacholine dose, the participant was requested to wash their mouth with 5 ml distilled water to recover saliva and expectorate the contents into a previously labeled storage container. This sample was concentrated through the use of a C-18 cartridge that retains the leukotrienes. The leukotrienes were then isolated and assayed by ELISA assay. Asthmatics were then defined as being predominantly leukotriene positive or negative based on multiple saliva samples isolated at differing methacholine test levels, and were defined as leukotriene based asthmatics, previously known as Responders, or non-leukotreine based asthmatics, previously known as Nonresponders or Middle asthmatics. Sputum was isolated through the use of isotonic saline and multiple inhalations with the use of a nebulizer in case it was necessary to use this fluid to determine leukotriene levels if saliva was not a viable fluid for use. At four, five or six hours after the pulmonary function test and isolation of saliva and sputum, the subjects returned to the metabolic lab and 5 ml whole blood was isolated by venapuncture over EDTA. Plasma was isolated by differential centrifugation and plasma IL-4, IL-16, and GM-CSF levels were ascertained by ELISA. While plasma was used for these assays, whole blood could also be used. If whole blood is utilized, it is preferable that an anticoagulant be included during isolation of the blood sample. Blood samples drawn at four, five and six hours post-methacholine challenge were examined to determine what would be the peak time for determination of plasma cytokine profiles.

### Results:

When a patient exhibited high leukotriene levels in response to methacholine challenge, they were determined to be a leukotriene based asthmatic. When a low or lack of production of leukotriene in saliva was exhibited in response to methacholine challenge, asthmatics were identified as cytokine based asthmatics. Plasma IL-4 and IL-16 profiles for plasma and saliva, and sputum GM-CSF profiles were then used to identify subjects as either a predominantly a IL-4 based asthmatic, a IL-16 based asthmatic, or a GM-CSF based asthmatic.

Table II illustrates the specific doses of Methacholine which were administered to induce a leukotreine response. Tables III-V, illustrate which cytokines were measured, and what body fluids were assayed.

**Table II:**

| Dose of Methacholine to Induce Leukotriene Response Cumulative Methacholine Dose in Units | | | | | | | | | | | |
|---|---|---|---|---|---|---|---|---|---|---|---|
| | Subject | | 0.125 | 1.370 | 3.875 | 8.875 | 18.875 | 31.38 | 81.38 | 206.38 | Cum |
| LK | 1 | | Pos | Pos | Pos | Neg | Pos | Neg | Pos | Pos | Pos |
| LK | 2 | | Neg | Pos | Pos | Pos | Pos | Pos | 0 | 0 | Pos |
| LK | 3 | | Neg | Neg | Neg | Neg | Neg | 0 | 0 | 0 | Neg |
| LK | 4 | | Pos | Pos | Pos | Pos | Pos | Pos | Neg | 0 | Pos |
| LK | 5 | | Pos | N/A | Pos | Pos | Pos | Pos | Pos | Pos | Pos |
| LK | 6 | | Neg | Pos | Pos | Pos | Pos | Pos | 0 | 0 | Pos |
| LK | 7 | | Neg | Neg | Neg | Neg | Neg | Pos | Neg | Neg | Neg |
| LK | 8 | | Neg | Neg | Neg | Neg | Pos | Pos | Neg | 0 | Neg |
| LK | 9 | | Neg | Pos | Neg | Neg | Neg | Neg | Neg | Pos | Neg |
| LK | 10 | | Neg | Neg | Neg | 0 | 0 | 0 | 0 | 0 | Neg |
| LK | 11 | | Neg | Neg | Pos | Neg | Neg | 0 | 0 | 0 | Neg |

| | | | | | | | | | | | |
|---|---|---|---|---|---|---|---|---|---|---|---|
| N/A - indicates that the test results were neither clearly negative, nor clearly positive. "0" indicates that the test was not run due to definitive reaction, or lack of reaction at lower dose. | | | | | | | | | | | |

**Table III:**

| *IL-4 levels in Response to* Progressive *Methacholine Challenge* | | | | | |
|---|---|---|---|---|---|
| | Subject | | | Plasma | Total |
| | | | | | |
| IL 4 | 1 | | | 0.00 | 0.00 |
| IL 4 | 2 | | | 73.75 | 73.75 |
| IL 4 | 3 | | | 71.25 | 71.25 |
| IL 4 | 4 | | | 15.60 | 15.60 |
| IL 4 | 5 | | | 15.60 | 15.60 |
| IL 4 | 6 | | | 42.00 | 42.00 |
| IL 4 | 7 | | | 12.60 | 12.60 |
| IL 4 | 8 | | | 61.25 | 61.25 |
| IL 4 | 9 | | | 78.75 | 78.75 |
| IL 4 | 10 | | | 42.00 | 42.00 |
| IL 4 | 11 | | | 6.00 | 0.00 |

**Table IV:**

| *IL-16 levels in Response to Progressive Methacholine Challenge* | | | | | |
|---|---|---|---|---|---|
| | Subject | | Sputum | Plasma | Sum |
| IL 16 | 1 | | 23.50 | 7.70 | 31.20 |
| IL 16 | 2 | | 57.00 | 29.00 | 86.00 |
| IL 16 | 3 | | 51.00 | 103.00 | 154.00 |
| IL 16 | 4 | | 24.50 | 63.00 | 87.50 |
| IL 16 | 5 | | 10.40 | 47.00 | 57.40 |
| IL 16 | 6 | | 29.00 | 26.00 | 55.00 |
| IL 16 | 7 | | 138.00 | 68.00 | 206.00 |
| IL 16 | 8 | | 143.00 | 55.50 | 198.50 |
| IL 16 | 9 | | 18.50 | 59.00 | 77.50 |
| IL 16 | 10 | | 105.50 | 70.50 | 176.00 |
| IL 16 | 11 | | 78.00 | 88.00 | 166.00 |

**Table V**

| *GM-CSF levels in Response to Progressive Methacholine Challenge* | | | | | | |
|---|---|---|---|---|---|---|
| | Subject | | Saliva | Sputum | Plasma | Total |
| GM-CSF | 1 | | 0 | 0 | 17.4 | 17.4 |
| GM-CSF | 2 | | 0 | 0 | 5.2 | 5.2 |
| GM-CSF | 3 | | 0 | 0 | 0 | 0 |
| GM-CSF | 4 | | 0 | 0 | 0 | 0 |
| GM-CSF | 5 | | 0 | 0 | 13.5 | 13.5 |
| GM-CSF | 6 | | 0 | 9 | 0 | 9 |
| GM-CSF | 7 | | 0 | 0 | 6.5 | 6.5 |
| GM-CSF | 8 | | 0 | 16.5 | 0 | 16.5 |
| GM-CSF | 9 | | 16.5 | 12.3 | 17.4 | 46.2 |
| GM-CSF | 10 | | 0 | 0 | 0 | 0 |
| GM-CSF | 11 | | 0 | 0 | 16.8 | 16.8 |

This test confirmed that responders, middle responders, and non-responders exhibit specific cytokine and leukotriene profiles, from which a specific asthmatic diagnosis may be determined, and accordingly, the most effective treatment protocol selected.

Table VI illustrates the various patient profiles which resulted from the above described studies:

| Test | Subject | | Sputum | Plasma | Sum | | M- 31.38 | M- 81.38 | M- 206.38 | Type | R/Nm/N |
|---|---|---|---|---|---|---|---|---|---|---|---|
| IL4 | 1 | | 0 | 0 | 0 | | | | | | |
| IL16 | 1 | | 23.5 | 7.7 | 31.2 | | | | | | |
| LK | 1 | | | | | | Neg | Pos | Pos | LT | Resp |
| GM-CSF | 1 | | 0 | 17.4 | 17.4 | | | | | | |
| | | | | | | | | | | | |
| IL4 | 2 | | 0 | 0 | 73.75 | | | | | | |
| | 2 | | 57 | 29 | 86 | | | | | | |
| I16 LK | 2 | | | | | | Pos | 0 | 0 | LT | Resp |
| GM-CSF | 2 | | 0 | 5.2 | 5.2 | | | | | | |
| | | | | | | | | | | | |
| IL4 | 3 | | 0 | 0 | 71.25 | | | | | | |
| IL16 | 3 | | 51 | 103 | 154 | | | | | | |
| LK | 3 | | | | | | 0 | 0 | 0 | IL-4 | Non |
| GM-CSF | 3 | | 0 | 0 | 0 | | | | | | |
| | | | | | | | | | | | |
| IL4 | 4 | | 0 | 0 | 15.6 | | | | | | |
| IL16 | 4 | | 24.5 | 63 | 87.5 | | | | | | |
| LK | 4 | | | | | | Pos | Neg | 0 | LT | Resp |
| GM-CSF | 4 | | 0 | 0 | 0 | | | | | | |
| | | | | | | | | | | | |
| IL4 | 5 | | 0 | 0 | 15.6 | | | | | | |
| IL16 | 5 | | 10.4 | 47 | 57.4 | | | | | | |
| LK | 5 | | | | | | Pos | Pos | Pos | LT | Resp |
| GM-CSF | 5 | | 0 | 13.5 | 13.5 | | | | | | |
| | | | | | | | | | | | |
| IL4 | 6 | | 0 | 0 | 42 | | | | | | |
| IL16 | 6 | | 29 | 26 | 55 | | | | | | |
| LK | 6 | | | | | | Pos | 0 | 0 | LT | Resp |
| GM-CSF | 6 | | 9 | 0 | 9 | | | | | | |
| | | | | | | | | | | | |
| IL4 | 7 | | 0 | 0 | 12.6 | | | | | | |
| IL16 | 7 | | 138 | 68 | 206 | | | | | | |
| LK | 7 | | | | | | Pos | Neg | Neg | IL-16 | Middle |
| GM-CSF | 7 | | 0 | 6.5 | 6.5 | | | | | | |
| | | | | | | | | | | | |
| IL4 | 8 | | 0 | 0 | 61.25 | | | | | | |
| IL16 | 8 | | 143 | 55.5 | 198.5 | | | | | | |
| LK | 8 | | | | | | Pos | Neg | 0 | IL-4 | Middle |
| GM-CSF | 8 | | 16.5 | 0 16.5 | | | | | | | |
| | | | | | | | | | | | |
| IL4 | 9 | | 0 | 0 | 78.75 | | | | | | |
| IL16 | 9 | | 18.5 | 59 | 77.5 | | | | | | |
| LK | 9 | | | | | | Neg | Neg | Pos | IL-4 | Non |
| GM-CSF IGM-CSF | 9 | | 12.3 | 17.4 | 46.2 | | | | | GH-CSF Based | |
| | | | | | | | | | | | |
| IL4 | 10 | | 0 | 0 | 42 | | | | | | |
| IL16 | 10 | | 105.5 | 70.5 | 176 | | | | | | |
| LK | 10 | | | | | | 0 | 0 | 0 | IL-16 | Non |
| GM-CSF | 10 | | 0 | 0 | 0 | | | | | | |
| | | | | | | | | | | | |
| IL4 | 11 | | 0 | 0 | 0 | | | | | | |
| IL16 | 11 | | 78 | 88 | 166 | | | | | | |
| LK | 11 | | | | | | 0 | 0 | 0 | IL-16 | Middle |
| GM-CSF | 11 | | 0 | 16.8 | 16.8 | | | | | | |

As is illustrated in Table VI, determination of leukotriene levels at specific methacholine doses, and subsequent determination of cytokine levels enables the clinician to diagnose asthmatics (e.g., leukotriene based, IL-4 based, IL-16 based, or GM-CSF based). These determinations provide the clinician with important criteria for selecting an appropriate course of therapeutic action.

### Discussion:

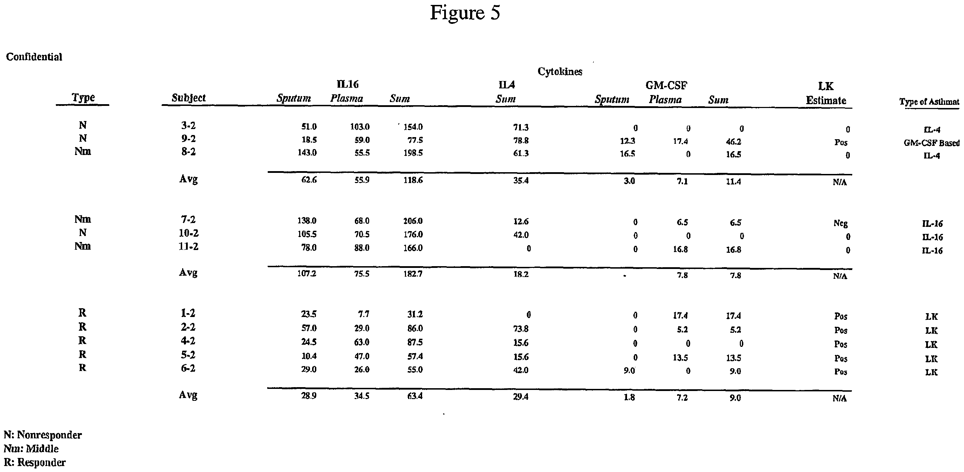

The results from the foregoing study are summarized in Figure 5. Patients which exhibit elevated levels of leukotrienes during or immediately following an asthmatic attack are considered leukotriene positive asthmatics, or responders. These patients are more likely to benefit from treatments which target leukotrienes. Patients which do not exhibit elevated levels of leukotrienes, but which do exhibit elevated levels of cytokines are considered cytokine based asthmatics, or middle responders and non-responders. These patients may be further differentiated as being IL-4 asthmatics, IL-16 asthmatics, or GM-CSF based, depending on which cytokine is most elevated. Accordingly, patients are most likely to respond to therapeutic agents medicines which specifically inhibit the leukotriene or cytokine that is most elevated during and immediately following an asthma attack.

Also, it has been determined that lower doses of Methacholine can be used to conduct a pulmonary function test, particularly in leukotriene based asthmatics. The ability to use lower levels of methacholine conveys two benefits. First it allows any physician to run a bronchoprovocation test, i.e. a test for asthma, in a clinical setting without the need to refer a patient to a hospital for the specific diagnosis. This allows more physicians to test for asthma and reduces the overall cost of the test when it can be conducted in a clinical setting instead of a hospital. Second, by using a lower dose of methacholine it reduces the likelihood that a full asthma episode will be induced while allowing for the induction of the release of cytokines and leukotrienes associated with asthma. This reduces the potential health risk to the patient, the medical liability to the physician, yet allows for the diagnosis and typing of asthma at a much lower risk to all involved.

Further, it has been determined that leukotrienes are most accurately measured immediately following an asthmatic attack or induction of a pulmonary function test, in saliva or sputum. Cytokines, however are best measured in blood or plasma, approximately four hours post episode. Identification of the foregoing clinical parameters aids the physician in the diagnosis and management of asthmatic patients.

## Claims

1. A method for determining whether an asthmatic patient is a leukotriene-based or cytokine-based asthmatic comprising:
a) contacting a biological sample from said patient with
(i) a plurality of first binding agents, said plurality of first binding agents comprising binding agents having respective binding affinity for each of IL-4, IL-16 and GM-CSF, and
(ii) a plurality of second binding agents, said plurality of second binding agents having respective binding affinity for each of leukotriene C4, leukotriene D4 and leukotriene E4,
thereby forming complexes;
b) contacting said complexes with at least one detectably labeled reagent having binding affinity for said first and second binding agents;
c) determining levels of said IL-4, IL-16, GM-CSF and leukotriene C4, leukotriene D4 and leukotriene E4 in said biological sample as a function of binding of said detectably labeled reagents.

2. The method of claim 1, wherein the plurality of first binding agents further comprises binding agents having respective binding affinity for IL-5 and/or IL-16.

3. The method of claim 1 or claim 2 wherein said biological sample is obtained following a pulmonary function test which comprises administration of an agent selected from the group consisting of methacholine, histamine, Manitol, adenosine, FELD (Cat dander), dust mite allergen, specific allergens, and any combination thereof.

4. The method of any one of claims 1 to 3, wherein said binding agents are monoclonal antibodies, and wherein said reagents are detectably labeled secondary antibodies having binding affinity for said monoclonal antibodies.

5. The method of any one of claims 1 to 4, wherein said biological sample is selected from the group consisting of saliva, sputum, blood, bronchial lavage, plasma, and any combination thereof.

6. The method of any one of claims 1 to 5 optionally further comprising augmentation of the sample with exogenous leukotriene to assess displacement of leukotriene present in said patient sample in a competitive binding assay, said exogenous leukotriene being detectably labeled.

7. The method of claim 3 wherein levels of said IL-4, IL-16, GM-CSF and leukotriene C4, leukotriene D4 and leukotriene E4are measured about four to six hours following administration of said pulmonary function test.

8. A kit for practicing the method of claim 1 comprising:
a plurality of monoclonal antibodies immunologically specific for IL-4, IL-16, GM-CSF, leukotriene C4, leukotriene D4 and leukotriene E4, a plurality of secondary antibodies which have affinity for said monoclonal antibodies, said secondary antibodies comprising a detectable label, and optionally a solid support.

9. The kit of claim 8, further comprising monoclonal antibodies immunologically specific for IL-5 and/or IL-13.

10. The kit of claim 8 or claim 9, further comprising methacholine.

11. The kit of claim 8 or claim 9, further comprising a detectably labeled leukotriene.

## Patentansprüche

1. Verfahren zur Bestimmung, ob ein Asthmapatient an Asthma auf Leukotrien-Basis oder Zytokin-Basis leidet, umfassend:
a) Kontaktieren einer biologischen Probe vom Patienten mit
(i) mehreren ersten Bindemitteln, wobei die mehreren ersten Bindemittel Bindemittel mit Bindungsaffinität für jeweils IL-4, IL-16 bzw. GM-CSF umfassen, und
(ii) mehreren zweiten Bindemitteln, wobei die mehreren zweiten Bindemittel Bindungsaffinität für jeweils Leukotrien C4, Leukotrien D4 bzw. Leukotrien E4 aufweisen,
wodurch Komplexe gebildet werden;
b) Kontaktieren der Komplexe mit zumindest einem detektierbar markierten Reagens mit Bindungsaffinität für das erste und zweite Bindemittel;
c) Bestimmen des Gehalts an IL-4, IL-16, GM-CSF und Leukotrien C4, Leukotrien D4 und Leukotrien E4 in der biologischen Probe als Funktion der Bindung der detektierbar markierten Reagenzien.

2. Verfahren nach Anspruch 1, wobei die mehreren ersten Bindemittel weiters Bindemittel mit Bindungsaffinität für IL-5 und/oder IL-16 umfassen.

3. Verfahren nach Anspruch 1 oder Anspruch 2, wobei die biologische Probe nach einem Lungenfunktionstest erhalten wird, der die Verabreichung eines Mittels umfasst, das aus der aus Metacholin, Histamin, Manitol, Adenosin, FELD (Katzenschuppen), Staubmilbenallergen, spezifischen Allergenen und behebigen Kombinationen daraus bestehenden Gruppe ausgewählt ist.

4. Verfahren nach einem der Ansprüche 1 bis 3, wobei die Bindemittel monoklonale Antikörper sind und wobei die Reagenzien detektierbar markierte sekundäre Antikörper mit Bindungsaffinität für die monoklonalen Antikörper sind.

5. Verfahren nach einem der Ansprüche 1 bis 4, wobei die biologische Probe aus der aus Speichel, Sputum, Blut, Bronchiallavageflüssigkeit, Plasma und beliebigen Kombinationen daraus bestehenden Gruppe ausgewählt ist.

6. Verfahren nach einem der Ansprüche 1 bis 5, das gegebenenfalls weiters die Ergänzung der Probe mit exogenem Leukotrien umfasst, um die Verdrängung von in der Patientenprobe vorhandenem Leukotrien in einem kompetitiven Bindungstest zu beurteilen, wobei das exogene Leukotrien detektierbar markiert ist.

7. Verfahren nach Anspruch 3, wobei der Gehalt an IL-4, IL-16, GM-CSF und Leukotrien C4, Leukotrien D4 und Leukotrien E4 etwa vier bis sechs Stunden nach der Durchführung des Lungenfunktionstests gemessen wird.

8. Set zur Ausführung des Verfahrens nach Anspruch 1, umfassend:
mehrere monoklonale Antikörper, die immunologisch spezifisch für IL-4, IL-16, GM-CSF, Leukotrien C4, Leukotrien D4 und Leukotrien E4 sind, mehrere sekundäre Antikörper, die Affinität zu diesen monoklonalen Antikörpern aufweisen, wobei die sekundären Antikörper eine detektierbare Markierung umfassen, und gegebenenfalls einen festen Träger.

9. Set nach Anspruch 8, das weiters monoklonale Antikörper umfasst, die immunologisch spezifisch für IL-5 und/oder IL-13 sind.

10. Set nach Anspruch 8 oder Anspruch 9, das weiters Metacholin umfasst.

11. Set nach Anspruch 8 oder Anspruch 9, das weiters detektierbar markiertes Leukotrien umfasst.

## Revendications

1. Procédé pour déterminer si un patient asthmatique est un asthmatique à leucotriènes ou à cytokines, consistant à :
a) mettre en contact un échantillon biologique dudit patient avec
(i) une pluralité de premiers agents de liaison, ladite pluralité de premiers agents de liaison comprenant des agents de liaison ayant une affinité de liaison respective pour chacun(e) parmi l'IL-4, l'IL-16 et le GM-CSF, et
(ii) une pluralité de deuxièmes agents de liaison, ladite pluralité de deuxièmes agents de liaison ayant une affinité de liaison respective pour chacun parmi le leucotriène C4, le leucotriène D4 et le leucotriène E4,
ce qui forme ainsi des complexes ;
b) mettre en contact lesdits complexes avec au moins un réactif marqué de manière détectable ayant une affinité de liaison pour lesdits premiers et deuxièmes agents de liaison ;
c) déterminer les taux desdits IL-4, IL-16, GM-CSF et leucotriène C4, leucotriène D4 et leucotriène E4 dans ledit échantillon biologique en fonction de la liaison desdits réactifs marqués de manière détectable.

2. Procédé selon la revendication 1, dans lequel la pluralité de premiers agents de liaison comprend en outre des agents de liaison ayant une affinité de liaison respective pour l'IL-5 et/ou l'IL-16.

3. Procédé selon la revendication 1 ou la revendication 2, dans lequel ledit échantillon biologique est obtenu suite à un test de la fonction pulmonaire qui comprend une administration d'un agent sélectionné dans le groupe consistant en la méthacholine, l'histamine, le mannitol, l'adénosine, FELD (phanère de chat), un allergène d'acarien, des allergènes spécifiques, et une quelconque combinaison de ceux-ci.

4. Procédé selon l'une quelconque des revendications 1 à 3, dans lequel lesdits agents de liaison sont des anticorps monoclonaux, et dans lequel lesdits réactifs sont des anticorps secondaires marqués de manière détectable ayant une affinité de liaison pour lesdits anticorps monoclonaux.

5. Procédé selon l'une quelconque des revendications 1 à 4, dans lequel ledit échantillon biologique est sélectionné dans le groupe consistant en de la salive, des expectorations, du sang, un lavage bronchique, du plasma, et une quelconque combinaison de ceux-ci.

6. Procédé selon l'une quelconque des revendications 1 à 5, consistant en outre à augmenter la quantité d'un leucotriène exogène dans l'échantillon afin d'évaluer le déplacement du leucotriène présent dans ledit échantillon du patient lors d'un essai de liaison compétitive, ledit leucotriène exogène étant marqué de manière détectable.

7. Procédé selon la revendication 3, dans lequel les taux desdits IL-4, IL-16, GM-CSF et leucotriène C4, leucotriène D4 et leucotriène E4 sont mesurés environ quatre à six heures après l'administration dudit test de la fonction pulmonaire.

8. Kit pour mettre en oeuvre le procédé selon la revendication 1, comprenant : une pluralité d'anticorps monoclonaux immunologiquement spécifiques pour l'IL-4, l'IL-16, le GM-CSF, le leucotriène C4, le leucotriène D4 et le leucotriène E4, une pluralité d'anticorps secondaires qui possèdent une affinité pour lesdits anticorps monoclonaux, lesdits anticorps secondaires comprenant un marqueur détectable, et facultativement un support solide.

9. Kit selon la revendication 8, comprenant en outre des anticorps monoclonaux immunologiquement spécifiques pour l'IL-5 et/ou l'IL-13.

10. Kit selon la revendication 8 ou la revendication 9, comprenant en outre de la méthacholine.

11. Kit selon la revendication 8 ou la revendication 9, comprenant en outre un leucotriène marqué de manière détectable.
